# EUROPEAN PATENT APPLICATION

(11) **EP 1 192 956 A2**
(43) Date of publication of application: **03.04.2002**
(21) Application number: 01308197.1
(22) Date of filing: 26.09.2001
(51) Int. Cl.: A61L 15/42

(54) **Absorbent article and barrier agent for absorbent article**

(30) Priority: 28.09.2000 JP 2000297141
(71) Applicant: UNI-CHARM CORPORATION, Kawanoe-shi Ehime-ken (JP)
(72) Inventor: Hisanaka, Takayuki, c/o Technical Center, Mitoyo-gun, Kanagawa-ken 769-1602 (JP); Miyazawa, Kiyoshi, c/o Technical Center, Mitoyo-gun, Kanagawa-ken 769-1602 (JP); Endo, Tomoko, c/o Technical Center, Mitoyo-gun, Kanagawa-ken 769-1602 (JP)
(74) Representative: Parry, Christopher Stephen

(57) **Abstract**

The present invention is accomplished to provide an absorbent article, such as a diaper and sanitary napkin, which can prevent a rash in the wearer and does not impart a sticky sensation to the wearer, and a barrier agent to be applied to the surface of an absorbent article. A barrier agent for an absorbent article containing powder for improving the tactile sensation incorporated therein or attached on the surface, and an absorbent article with the barrier agent attached on the surface are provided.

## Description

### TECHNICAL FIELD

The present invention relates to an absorbent article such as a diaper, sanitary napkin, or the like which can reduce dermatitis (rash) brought when such an absorbent article is worn and imparts no sticky sensation to the wearer, and to a barrier agent to be applied to the surface of the absorbent article. More specifically, the present invention relates to an absorbent article such as a diaper, sanitary napkin, or the like which can prevent contact-type dermatitis (rash) from being produced due to a chemical stimulation from body fluids such as feces, urine, menstrual bleeding, and the like or due to physical stimulation of the skin by friction with respect to the surface materials of the absorbent article, and which does not impart a sticky sensation to the wearer. The present invention also relates to a barrier agent to be applied to the surface of the absorbent article for the said purpose.

### BACKGROUND ART

A problem with an absorbent article such as a diaper, sanitary napkin, or the like is dermatitis produced by attachment of feces, urine, menstrual bleeding, and the like to the skin, or stimulation of the skin by chemicals such as a surfactant used for improving the surface properties of the absorbent article, or contact-type dermatitis produced by a physical stimulation of the skin due to friction between the skin and the surface material of the absorbent article.

In addition, a synergistic action of these chemical and physical stimulators increases stimulation of the skin and swells the skin, resulting in an increase in the coefficient of friction between the surface material of the absorbent article and the skin.

In order to prevent such contact dermatitis (rash), it is important to thoroughly wipe off body fluids attached to the skin when an absorbent article is replaced, thereby maintaining the skin clean. However, it is impossible to completely avoid contact of body fluids with the skin which may occur during the period after a body fluid has become attached to the skin and before the absorbent article is replaced. The effort of frequently replacing the absorbent article can shorten the period of time during which the body fluids are in contact with the skin. However, there is a limitation to the number of times of replacement.

One method for preventing contact dermatitis is to apply a cream, oil, or the like to the skin of the wearer such as an infant. Although it is possible to apply a cream, oil, or the like to the buttocks or the anal region of the wearer when the diaper is replaced, application of such an agent each time the diaper is replaced is not only troublesome, but also may create a problem associated with sanitation. That is, the diaper wearer who has weak skin such as an infant or an aged person wearer may be infected with bacteria via the fingers of the care person or helper who applies the cream to the diaper wearer.

It has been tried to solve these problems by applying a skin care substance to the surface of absorbent article. As one of these trails Japanese Patent Application Laid-open No. 1265/1990 discloses a method of using a pH-adjusting agent with an absorbent article for incontinence. This method has been developed based on the finding that the cause of diaper rash is ammonia produced by bacteria from feces or by the action of an enzyme from urine. The ammonia increases the pH of the skin and causes a rash to occur. However, a rash caused by a substance other than ammonia cannot be prevented by a pH-adjusting agent.

Japanese Patent Applications Laid-open No. 509895/1998, No. 509896/1998, No. 510082/1999, and No. 510416/1999 disclose disposable absorbent articles with a specific lotion applied to the surface. The lotion comprises an emollient agent for improving lubricity and an immobilizing agent to attach the emollient agent to the surface of the absorbent articles. The emollient agent in these absorbent articles melts by the wearer's body temperature and becomes attached to the skin. The emollient agent transferred on the skin surface is expected to reduce stimulation of the skin by body fluids and protect the skin from physical stimulation by increasing lubricity between the surface of the absorbent article and the skin.

However, experiments by the present inventors have revealed the absorbent articles disclosed by Japanese Patent Applications Laid-open No. 509895/1998, No. 509896/1998, No. 510082/1999, and No. 510416/1999 cannot sufficiently prevent a rash in the diaper wearers.

In addition, these methods of forming an oily emollient barrier layer on the skin surface impart a sticky or slimy sensation because oily components in the barrier layer attach to the skin of the wearer. Therefore, the wearing sensation of the wearer or the sensation of touch to the absorbent article is impaired.

### SUMMARY OF THE INVENTION

An object of the present invention is therefore to solve the above problems in those prior arts. Specifically, an object of the present invention is to provide an absorbent article such as a diaper, sanitary napkin, or the like which can prevent a rash in the wearer and does not impart sticky sensation to the wearer, and to provide a barrier agent to be applied to the surface of such an absorbent article.

More specifically, an object of the present invention is to provide a barrier agent to be applied to the surface of an absorbent article, which can prevent contact-type dermatitis (rash) due to chemical stimulation by body fluids, surfactants, etc. or a physical stimulation of the skin by friction with the surface of the absorbent article and does not impart a sticky sensation to the wearer, and to provide an absorbent article to which the barrier agent has been applied.

As a result of extensive studies for attaining the above object, the present inventors have found that, in addition to chemical stimulation and physical stimulation by the above body fluids, amino acids and sebum which are spodogenous materials of body fluids remaining on the skin are a cause of contact-type dermatitis (rash). This finding has led to the completion of the present invention. More specifically, the inventors of the present invention have found that in the absorbent articles disclosed by Japanese Patent Applications Laid-open No. 509895/1998, No. 509896/1998, No. 510082/1999, and No. 510416/1999 spodogenous materials of body fluids attached to the skin are covered by the lotion composition and easily deteriorate into substances which stimulate the skin and induce rash. The present inventors have conceived an idea of adding an oxidant to the barrier composition to prevent the spodogenous materials from being oxidized and deteriorating and completed the present invention. Furthermore, the inventors of the present invention have found that a sticky or slimy sensation imparted to the wearer due to attachment of an oily emollient of the barrier agent to the skin surface when using an absorbent article containing the above lotion composition as a barrier agent can be reduced by incorporating a powder for the improvement of tactile sensation into the barrier agent or attaching such a powder to the surface of the barrier agent. This finding has also led to the completion of the present invention.

Therefore, the present invention relates to:
(1) A barrier agent for an absorbent article, wherein a powder for the improvement of tactile sensation is mixed therein or attached to the surface,
(2) The barrier agent for an absorbent article described in (1) above, wherein the powder is one or more powders selected from the group consisting of a natural inorganic powder, synthetic inorganic powder, organic powder, metallic soap, and synthetic polymer powder,
(3) The barrier agent for an absorbent article described in (1) or (2) above further comprising an oil soluble antioxidant,
(4) The barrier agent for an absorbent article described in (3) above, wherein the antioxidant is one or more vitamins selected from the group consisting of vitamin A group, vitamin B group, vitamins D group, vitamin E group, and vitamin K group,
(5) The barrier agent for an absorbent article described in (1) to (4) above further comprising an emollient agent or immobilizing agent, or both,
(6) The barrier agent for an absorbent article described in (1)-(5) above, wherein the emollient agent is an oily substance which is liquid or semi-solid at 25°C,
(7) The barrier agent for an absorbent article described in (6) above, wherein the emollient agent is a fatty acid glyceride.
(8) The barrier agent for an absorbent article described in (1)-(7) above, wherein the immobilizing agent has a melting point of 40-90°C and a surface tension of 25-45 dyn/cm at 20°C,
(9) The barrier agent for an absorbent article described in (8) above, wherein the immobilizing agent is one or more compounds selected from the group consisting of a fatty acid glyceride, paraffin wax, and a higher alcohol, and
(10) An absorbent article to the surface of which a barrier agent described in any one of (1) to (9) above has been applied.

Causing the powder for the improvement of tactile sensation to be present on the surface of the absorbent article according to the present invention not only improves the sticky or slimy sensation imparted to the wearer, but also can prevent the wearer's or the purchaser's willingness of buying the absorbent article from being reduced due to unfavorable sultry image of the sticky or slimy sensation, particularly in summer during which the temperature and humidity are high. In addition, improvement of sticky sensation also improves the skin conditions of the wearer.

Application of the barrier agent of the present invention to the surface of an absorbent article brings about not only the effect of not imparting a sticky sensation to the wearer, but also the effect of preventing generation of a skin rash, when the absorbent article is worn for a long period of time.

### DETAILED DESCRIPTION OF THE INVENTION

The present invention will be explained in more detail in the following description, which is not intended to be limiting of the present invention.

The absorbent article in the present invention includes disposal diapers, sanitary napkins, vaginal discharge liners, incontinence shorts, training pants, diaper holders, and the like.

The barrier agent of the present invention may be applied to the contacting area of these absorbent articles with the skin, for example, the top sheet, the three-dimensional gathers, the side flap, the waist gathers, and so on. Application to the top sheet to which feces or urine frequently become attached is particularly preferable.

The powder preferably used for the improvement of tactile sensation in the present invention is one or more powders elected from the group consisting of a natural inorganic powder such as pulverized fine particles of clay minerals, synthetic inorganic powder, organic powder, metallic soap, and synthetic polymer powder. If the average particle diameter of the powder is too small, the barrier agent cannot effectively prevent a sticky sensation; if too large, on the other hand, the particles impart a gritty sensation. For these reasons, the average particle diameter is preferably 0.05-50 µm, and more preferably 0.5-25 µm.

Typical natural inorganic powders are pulverized particles of clay minerals such as talc, kaolin, sericite, and mica.

Calcium carbonate, magnesium carbonate, silicic anhydride, zinc oxide, and titanium oxide can be given as examples of synthetic inorganic powder.

As a synthetic organic powder, amino acid-based powder is preferable, and N-lauroyl-L-lysine is particularly preferable.

As a synthetic polymer powder, polyethylene powder, nylon powder, polyacrylic acid powder, cross-linking poly styrene powder, reticular methyl siloxane polymer powder, and methacrylic resin powder, and the like can be given.

As a metallic soap, fatty acid salt of aluminum, zinc, magnesium, calcium, or the like which imparts only a slight stimulation to the skin and is used as cosmetic powders can be used. Specific examples include aluminum stearate, zinc stearate, magnesium stearate, calcium stearate, zinc palmitate, zinc myristate, magnesium myristate, zinc laurate, and zinc undecylenate.

An oil soluble antioxidant which is mutually soluble with an oily emollient agent is preferable as the antioxidant. As compounds which are oil soluble and have an antioxidant function, vitamins such as vitamin A group, vitamin B group (riboflavin tetrabutyrate, riboflavin tetranicotinate, etc.), vitamin D group, vitamin E group, and vitamin K group, BHT (2,6-di-t-butyl-p-cresol), BHA (butylated hydroxyanisole), and propyl gallate can be given.

These antioxidant agents can be used either individually or in combination of two or more.

Of the above antioxidant agents, vitamins are particularly preferable. Vitamins are also preferable due to the high degree of safety with respect to human body and few side effects. In addition, vitamins are widely used in articles coming directly into contact with the skin, such as cosmetics, and are known to exhibit the required effect when directly attached to skin.

Vitamins in the present invention include vitamins and derivatives thereof. Vitamin E group is particularly preferred compounds exhibiting the effect of the present invention and includes vitamin E (tocopherol), isomers and derivatives thereof such as DL-α-tocopherol, DL-α-tocopherol acetate, DL-α-tocopherol succinate, calcium DL-α-tocopherol succinate, and the like. Of these, vitamin E is particularly preferable due to the high antioxidation effect.

The emollient agent in the present invention refers to a lubricity promoting substance that has a function to soften, relax, or cover the skin, and to make the skin flexible, smooth, moisturized, and clean. An oily substance which is liquid or semi-solid at ambient temperature (25°C) is preferable as an emollient agent.

Although any known emollient agents (see Japanese Patent Applications Laid-open Nos. 509895/1998, 509896/1998, 510082/1999, and 510416/1999, for example) can be used, useful substances are petroleum hydrocarbons, oils and fats of animal or vegetable origin, wax of animal or vegetable origin, fatty acid esters, alkylethoxylates, fatty acid ester ethoxylates, fatty alcohols, polysiloxanes, and the like.

Specific examples of vegetable oils include drying oils such as grape seed oil, safflower oil, and soybean oil, semidrying oils such as sesame oil, corn oil, cottonseed oil, rapeseed oil, and sunflower oil, and nondrying oils such as coconut oil, avocado oil, almond oil, olive oil, sasanqua oil, camellia oil, persic oil, castor oil, and peanut oil.

As vegetable fats, cacao butter, palm oil, palm kernel oil, macadamia nut oil, Japan tallow, coconut butter, and the like can be given.

As vegetable waxes, carnauba wax, candellila wax, jojoba oil, and the like can be given.

As animal fats and oils, turtle oil, mink oil, egg yolk oil, beef tallow, lard, sardine oil, shark liver oil, herring oil, and the like can be given.

As animal waxes, whale wax, bees' wax, lanolin, and the like can be given.

As examples of hydrocarbon compounds, dialkyl carbonate having 14-15 carbon atoms, petrolatum (vaseline), paraffin, squalane, pristane, ozokerite, sericin, micro-crystallin wax, and the like can be given.

These fats and oils of animal or vegetable origin, waxes of animal or vegetable origin, or hydrocarbon compounds may contain hydrogenated unsaturated moieties.

These compounds may be used either individually or in combination of two or more as an emollient.

A preferable emollient agent is a fatty acid ester, and a fatty acid glyceride is particularly preferable. Any fatty acid glyceride of a monoglyceride, diglyceride, or triglyceride may be useful. The number of carbon atoms of the fatty acid moiety thereof may be 2-35, and preferably 8-22. A particularly preferable fatty acid glyceride is a coconut oil fatty acid triglyceride. Coconut oil fatty acid triglyceride is highly safe with respect to human body and exhibits almost no side effects. In addition, triglycerides are compounds constituting sebum, which is known to exhibit an emollient effect on the human skin surface. Therefore, selection of a triglyceride is particularly preferable.

The immobilizing agent is a substance for fixing and immobilizing the emollient agent on the surface of an absorbent article, is soluble or dispersible in oils or fats, and, if mixed with an emollient agent, increases the viscosity of the emollient agent, making the emollient agent easily applied to the surface of the absorbent article. The immobilizing agent preferably has a melting point of 40-90°C and a surface tension of 25-45 dyne/cm² at 20°C.

Any immobilizing agents disclosed in the aforementioned Japanese Patent Applications Laid-open No. 509895/1998, No. 509896/1998, No. 510082/1999, and No. 510416/1999 can be used. Preferable immobilizing agents are oils and fats of animal or vegetable origin, fatty acid esters, paraffin wax, higher alcohols, and ethoxylated alcohols, which are highly safe for human body and exhibit almost no side effects. Particularly preferable compounds are fatty acid glycerides, esters of C₁₂₋₂₂ fatty acid and dextrin, paraffin wax, and higher alcohols having a melting point of 40-90°C.

As the immobilizing agent or the emollient agent, as described above, oils and fats of animal or vegetable origin can be used.

Coconut oil, cacao butter, cottonseed oil, peanut oil, soybean oil, olive oil, shea nut oil, and the like as oils and fats of vegetable origin, and beef tallow, lard, mutton tallow, and the like as oils and fats of animal origin may be used without special treatment. However, the properties such as melting point of those natural fats and oils are unstable, and it may be difficult to control the properties for the purpose.

For the reason, preferably raw fats and oils are hydrolyzed to fatty acids and glycerol, and the fatty acids obtained are separated and purified to desirable fatty acids by distillation. The fatty acids and glycerol obtained are composed to glycerol ester of fatty acid again. Said glycerol ester of fatty acid has properties controlled suitable for the immobilizing agent or the emollient agent, and thus is preferable for use to these agents.

The fatty acid glyceride may be any one of a monoglyceride, diglyceride, or triglyceride, the number of carbon atoms of the fatty acid moiety thereof is preferably 2-35, and particularly preferably 8-22. Most preferable glycerides are the monoglyceride of stearic acid and the monoglyceride of lauric acid. Fatty acid dextrin esters include, for example, dextrin palmitate, dextrin stearate, dextrin behenate, dextrin myristate, dextrin coconut oil fatty acid ester, and dextrin laurate.

Paraffin wax having 16-40 carbon atoms, a melting point of 40-90°C, and a specific gravity at 20°C of 0.88-0.92 is preferable.

A higher alcohol having a carbon atom content of 14-32, particularly 16-22, is preferable. A particularly preferable higher alcohol is stearyl alcohol.

These immobilizing agents can be used either individually or in combination of two or more.

The barrier agent of the present invention may comprise an antioxidant and an emollient agent; an antioxidant and an immobilizing agent; or an antioxidant, an emollient agent, and an immobilizing agent.

In addition to the powder for improving tactile sensation, antioxidant, emollient agent, and immobilizing agent, the barrier agent of the present invention may optionally comprise any components, which are commonly used for improving the performances of the absorbent articles. Such optional components may include an anti-inflammatory agent, pH-adjusting agent, and surfactant to improve absorbency. An antibacterial agent and humectant may also be added.

The anti-inflammatory agent which can be used includes naturally occurring anti-inflammatory agents such as peony, ogon, hypericum, chamomile, glycyrrhiza, peach leaf, mugwort, and perilla frutescens extract, and synthetic anti-inflammatory agents such as allantoin and dipotassium glycyrrhizinate.

A pH-adjusting agent may be added to maintain the skin in a weakly acidic condition. Malic acid, succinic acid, citric acid, tartaric acid, lactic acid, and the like can be used as the pH-adjusting agent.

As a surfactant for improving absorbency, surfactants imparting a low irritation to the skin such as a sucrose fatty acid ester can be selected.

The powder for improving tactile sensation used in the present invention may be either mixed with the barrier agent which comprises an antioxidant, emollient agent, immobilizing agent, and the like or attached to the surface of the barrier agent.

Specific examples are as follows.
(1) The powder for improving tactile sensation is mixed with the three-component barrier agent comprising an antioxidant, emollient agent, and immobilizing agent, and a single layer of the resulting mixture is formed on the surface of the absorbent article. In this instance, the ratio of the total weight of the emollient agent and antioxidant to the weight of the immobilizing agent is 5-95:95-5, and preferably 30-70:70-30, and the amount of powder is 0.001-70% by weight, preferably 0.01-40% by weight, of the three-component barrier agent. The amount of the barrier agent in which the powder has been incorporated per surface area of the absorbent article is 0.5-50 g/m², and preferably 1-30 g/m².
(2) A single layer of a three-component barrier consisting of an antioxidant, emollient agent, and immobilizing agent is formed on the surface of the absorbent article. In this instance, the ratio of the total weight of the emollient agent and antioxidant to the weight of the immobilizing agent is 5-95:95-5, and preferably 30-70:70-30, and the amount of the barrier agent per surface area of the absorbent article is 0.5-50 g/m², and preferably 1-30 g/m². Then, the powder for improving tactile sensation in the amount of 0.001-70% by weight, preferably 0.01-40% by weight, is attached to the skin-contacting surface of the three-component barrier agent.
(3) The barrier agent may be present on the surface of the absorbent article as a multi-layer.

In this instance, a preferable method is (i) forming a layer of a two-component mixture containing an emollient agent and immobilizing agent on the absorbent article, and causing a three-component mixture containing an emollient agent, antioxidant, and immobilizing agent to be present on the surface of that layer coming in direct contact with the skin, or (ii) forming a layer of a two-component mixture containing an emollient agent and immobilizing agent on the absorbent article, and causing a two-component mixture containing an antioxidant and immobilizing agent to be present on the surface of that layer coming in direct contact with the skin.

In either (i) or (ii), the powder for improving tactile sensation is mixed with the mixtures for both the layer on the absorbent article side and the layer on skin-contacting side, or the powder is mixed only with the mixture for the layer on the skin-contacting side. Alternatively, the powder may be attached to the surface of the skin-contacting side.

Such a multi-layer structure allows a greater proportion of antioxidant to be present on the surface coming into contact with the skin so that the antioxidant may adhere to the skin before the emollient agent covers spodogenous materials attached to the skin, thereby more increasing the effect of the present invention. In addition, because antioxidants are expensive materials, obtaining the target effect by using as small an amount of antioxidant as possible is desirable. In this respect, these methods of incorporating a larger amount of an antioxidant in the layer in contact with the skin are preferable.

In the above method (i) or (ii), the ratio of the emollient agent to immobilizing agent in the two-component mixture of the absorbent article side is 5-95:95-5, and preferably 30-70:70-30, and the amount of the mixture per surface area of absorbent article is 0.25-25 g/m², and preferably 0.5-15 g/m².

In the above method (i), the ratio of the total weight of the emollient agent and antioxidant to the weight of the immobilizing agent in the three-component mixture coming in contact with the skin is 5-95:95-5, and preferably 30-70:70-30, and the amount of the mixture per surface area of absorbent article is 0.25-25 g/m², and preferably 0.5-15 g/m².

In the above method (ii), the ratio of the antioxidant to immobilizing agent in the two-component mixture coming in contact with the skin is 5-95:95-5, and preferably 30-70:70-30, and the amount of mixture per surface area of absorbent article is 0.25-25 g/m², and preferably 0.5-15 g/m².

In either (i) or (ii) above, the mixing amount of the powder is 0.001-70% by weight, and preferably 0.01-40% by weight of the amount of the barrier agent. In the case of attaching the powder to the surface, the amount of 0.001-70% by weight, and preferably 0.01-40% by weight of the powder is also applied to the surface.

The application area of barrier agent per total area of the absorbent article in contact with the skin is 5-90%, and preferably 10-70%, either when the barrier agent is present in single layer as described in (1) and (2) above or present in multi-layers as described in (3) above.

A common application method may be employed for applying the barrier agent to the surface material of absorbent article.

When applying the barrier agent to the liquid-permeable surface material, the area to which the barrier agent has been applied exhibits decreased permeability. In such a case, both a hydrophilic area and the applied area must be present on the sheet surface. This is preferably accomplished by patterning the area being applied the barrier agent, and a gravure application method or flexography application method for the printing technique can be applicable therefor. In addition, an extrusion method, slot method, and spray method which are applications of a thermosensible adhesive application technique may also be used. A multi-layer can also be produced by these application methods.

The powder is either mixed with the barrier agent and applied to the surface of the absorbent article or attached to the surface of the absorbent article after application of the barrier agent. As the method of attaching the powder, (i) a method of mixing the powder with air and injecting to the substrate through nozzles, (ii) a method of feeding the powder to a cylindrical roller or a continuous belt and transcripting the powder onto the substrate, (iii) a method of feeding the powder directly onto the substrate, (iv) a method of causing the substrate to pass through a coating machine in which electrostatic powder is floating, and the like can be given.

The barrier agent of the present invention is applied to the surface of absorbent article coming in contact with the skin. Usually, this agent is applied to a top sheet. There are no specific limitations to the object to which the barrier agent is applied. Usually, such an object is a permeable sheet-like material such as a non-woven fabric or permeable porous film.

The non-woven fabric used in the absorbent article is made of 1-5 d fiber and has a density of 10-50 g/m². Fibers for non-woven fabric may be synthetic fiber such as polyolefin and polyester, semi-synthetic fiber such as rayon, or natural fiber such as cotton, pulp, and silk.

Permeable porous films usable in the present invention are made from thermoplastics by extrusion, followed by boring by heated needles, embossing, hot blast, or the like. Polyethylene (density: 0.86-1.1 g/cm³), polypropylene (density: 0.89-1.2 g/cm³), and the like can be used either individually or in combination (and either in single layer or multi-layer) as the thermoplastics for porous films.

A permeable sheet material must permit body fluids to permeate and must withstand 0-300 mm H₂O of water pressure according to the JIS L1092 (Test method for water resistance of textiles, water resistance test method A (low water pressure method)). In addition, the permeable sheet material must have gas permeability to permit water vapor to pass from the surface material to the absorbent in the range of 5-700 cm³/cm²/sec according to the JIS L1906 (Test methods for non-woven fabrics made of filament yarn, Frazier type permeameter test method).

The barrier agent of the present invention can be applied not only to absorbent articles, but also to skin care dry wipes such as a wound protection sheet, tissue paper, toilet paper, and the like, or skin care wet wipes such as wet tissue, and the like to prevent contact dermatitis (a rash) due to the use of these wipes. When applying to wet wipes, the barrier agent of the present invention is first applied to the surface of the wipes, followed by impregnation of an aqueous type drug solution. Wet wipes have both of an oily region and an aqueous region on the surface. The wet wipes not only can prevent inflammation due to an aqueous anti-inflammatory agent and remove aqueous soiled material by the aqueous region, but also can prevent contact dermatitis (a rash) due to the action of an oily barrier which is caused to adhere to the skin when the skin is wiped by the wet wipes.

### EXAMPLES

The present invention will be explained by way of examples in the following description, which is not intended to be limiting of the present invention.

### (Example 1)

A barrier agent was prepared by melting a mixture of coconut oil fatty acid triglyceride as an emollient agent, stearyl alcohol as an immobilizing agent, vitamin E as an antioxidant, and talc as powder for improving tactile sensation at a weight ratio of 45.0:45.0:0.5:0.5. This barrier agent was applied to the surface material of a diaper in a stripe pattern in an amount of 20 g/m².

The diaper comprising a top sheet having high liquid permeability, an absorber made from pulp and a high water-absorptive resin capable of adsorbing and retaining body fluid, and a back sheet of polyethylene film. This was an assembling-type diaper to be fixed to the trunk using an adhesive tape. A 25 g/m² non-woven fabric to which polyolefin fiber had been melt-adhered was used as the top sheet. The previously prepared barrier agent described above was melted by heating and applied to the continuously-supplied non-woven fabric for top sheet using a coater. After cooling a diaper was manufactured by assembling the top sheet to which the barrier agent has been applied, the absorber, and the back sheet.

### [Test for tactile sensation]

The diaper was applied to an infant. After five hours, the tactile sensation on the surface of the diaper and the buttocks was evaluated and rated according to the following five level criteria.
Level 1: Very sticky
Level 2: Slightly sticky
Level 3: Not sticky
Level 4: Slightly Smooth
Level 5: Very smooth

As a result, the tactile sensation of the diaper surface was level 4, and that of the infant buttocks was level 3.

### [Odor of oxidized fats and oils]

Five hours after the infant had worn the diaper, the odor was sensorially determined.

### [Oxidation of fats and oils]

Five hours after the infant had worn the diaper, sebum on the skin surface was analyzed according to the following skin surface sebum analyzing method.
(i) 0.4 mg of sebum was collected from the skin surface in the diaper attached area using cotton impregnated a 1 : 1 mixture solvent of acetone and ether.
(ii) The sebum was extracted with ether and the extract was dried in a reduced pressure at 30°C or less with an aspirator while flowing nitrogen gas.
(iii) 0.4 mg of the dry material obtained in (ii) above was dissolved in 100 µl of 1-butanol. 10 µl of the solution was injected into a CL (chemiluminescence)-HPLC system. Operating conditions of the CL-HPLC system were as follows ;
   Column: CAPCELL-PAK C18 (25 cm × 4.6 mm)
   Column temperature: 25°C
   Solvent: methanol
   Luminescence indication solvent: 10 µg/ml cytochrome C + 1.0 µg/ml borate buffer (pH=9.3)
   Flow rate: 1.1 ml/min. and
   Detector: Chemiluminescence Analyzer
(iv) The fats and oils were judged to have been oxidized when a peak was observed as a result of the analysis.

### [Test for evaluating the state of the skin]

The diaper was worn by a healthy infant having no erythema or the like in the external genital area. The diaper was replaced eight times every day for one week. The state of the skin was observed by the naked eye immediately after the diaper was taken off. As a result, no change in the skin conditions was observed. The results are summarized in Table 2.

### (Example 2)

The same experiment as in Example 1 was carried out, except that the powder was attached to the surface of the barrier agent instead of mixing with the barrier agent. The results of the tactile sensation indicated that the diaper surface sensation was level 5 and the tactile sensation on the infant buttocks was level 4. No odor of oxidized oils and fats was sensed, no oxidation of oils and fats was confirmed, and no change in the skin conditions was observed. The results are summarized in Table 2.

### (Example 3)

The experiment was carried out in the same manner as in Example 1, except for using zinc stearate as the powder for improving the tactile sensation. The results are shown in Table 2.

### (Example 4)

The experiment was carried out in the same manner as in Example 2, except for using zinc stearate as the powder for improving the tactile sensation. The results are shown in Table 2.

### (Example 5)

The experiment was carried out in the same manner as in Example 1, except for using stearic acid monoglyceride as the immobilizing agent. The stearic acid monoglyceride used here was a re-composed ester of glycerol and stearic acid. The stearic acid was obtained as stearic acid fraction by distillation of fatty acids that was separated from glycerol after hydrolyzing beef tallow. The results are shown in Table 2.

### (Example 6)

The experiment was carried out in the same manner as in Example 4, except for using stearic acid monoglyceride as the immobilizing agent. The same stearic acid monoglyceride was used as in Example 5. The results are shown in Table 2.

### (Example 7)

The experiment was carried out in the same manner as in Example 1, except for using lauric acid monoglyceride as the immobilizing agent. The lauric acid monoglyceride used here was a re-composed ester of glycerol and lauric acid. The laustearic acid was obtained as lauric acid fraction by distillation of fatty acids that was separated from glycerol after hydrolyzing beef tallow. The results are shown in Table 2.

### (Example 8)

The experiment was carried out in the same manner as in Example 4, except for using lauric acid monoglyceride as the immobilizing agent. The same lauric acid monoglyceride was used as in Example 7. The results are shown in Table 2.

### (Comparative Example 1)

The experiment was carried out in the same manner as in Example 1, except for using a barrier agent containing no powder and containing coconut oil fatty acid triglyceride, stearyl alcohol, and vitamin E at a weight ratio of 47.5:47.5:5.0. The results were level 1 for both the diaper surface and the infant buttocks, indicating the surfaces were very sticky. No change in the skin conditions was observed.

### (Comparative Example 2)

The experiment was carried out in the same manner as in Example 1, except for using a barrier agent containing no antioxidant nor powder, and containing coconut oil fatty acid triglyceride and stearyl alcohol at a weight ratio of 50.0:50.0. The results were level 1 for both the diaper surface and the infant buttocks, indicating that the surfaces were very sticky. An odor of oxidized oils and fats was sensed, oils and fats were oxidized, and slight erythema was observed on the skin.

The results of Examples 1-8 and Comparative Examples 1 and 2 are summarized in Tables 1 and 2.

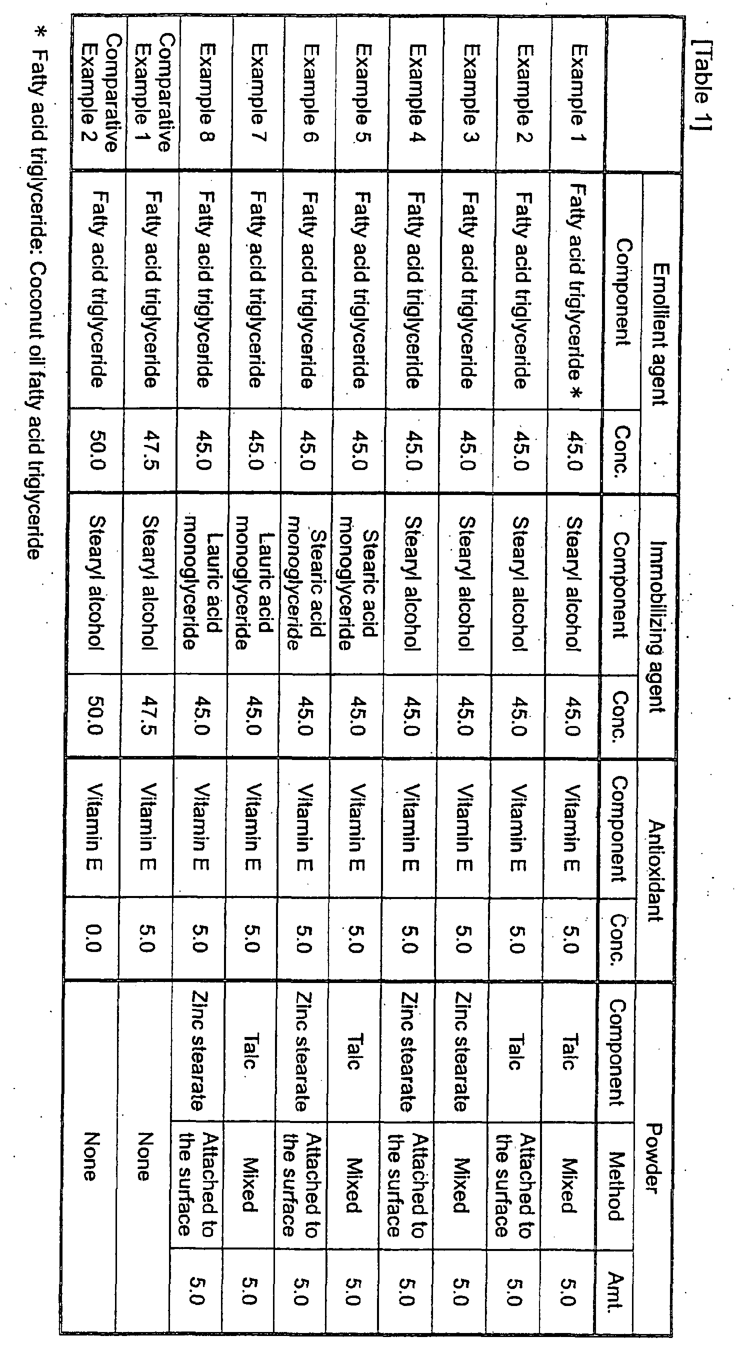

**[Table 2]**

| | Diaper surface | Infant buttocks | Odor of oils/fats | Oxidation of oils/fats | Skin conditions |
|---|---|---|---|---|---|
| Example 1 | Level 4 | Level 3 | Not sensed | Not oxidized | No change |
| Example 2 | Level 5 | Level 4 | Not sensed | Not oxidized | No change |
| Example 3 | Level 4 | Level 3 | Not sensed | Not oxidized | No change |
| Example 4 | Level 5 | Level 4 | Not sensed | Not oxidized | No change |
| Example 5 | Level 3 | Level 4 | Not sensed | Not oxidized | No change |
| Example 6 | Level 4 | Level 5 | Not sensed | Not oxidized | No change |
| Example 7 | Level 3 | Level 4 | Not sensed | Not oxidized | No change |
| Example 8 | Level 4 | Level 5 | Not sensed | Not oxidized | No change |
| Comparative Example 1 | Level 1 | Level 1 | Not sensed | Not oxidized | No change |
| Comparative Example 2 | Level 1 | Level 1 | Sensed | Oxidized | Slight erythema |

It can be seen from the results shown in Tables 1 and 2 that the tactile sensation of the wearer can be improved in an absorbent article applied the barrier agent by adding powder into the barrier agent, and change in the state of the skin of wearer can also be avoided. Though beef tallow was used as raw material of stearic acid monoglyceride and lauric acid monoglyceride in Examples 5-8, such a raw material should not be limited to beef tallow and other raw materials such as coconut oil or the like may be used and obtained same effects.

### INDUSTRIAL APPLICABILITY

The barrier agent for an absorbent article of the present invention, wherein a powder for the improvement of tactile sensation is mixed therein or attached to the surface, is expected excellent effects that the sticky or slimy sensation imparted to the wearer is significantly improved. The present invention also includes the absorbent article applied the barrier agent. Therefore, the present invention is very useful as an absorbent article such as disposal diapers, sanitary napkins, vaginal discharge liners, incontinence shorts, training pants, diaper holders, and the like, and also useful as a barrier agent for applying on the surface thereof.

## Claims

1. A barrier agent for an absorbent article, wherein a powder for the improvement of tactile sensation is mixed therein or attached to the surface.

2. The barrier agent for the absorbent article according to claim 1, wherein the powder is one or more members selected from the group consisting of a natural inorganic powder, synthetic inorganic powder, organic powder, metallic soap, and synthetic polymer powder.

3. The barrier agent for the absorbent article according to claim 1 or 2, further comprising an oil soluble antioxidant.

4. The barrier agent for the absorbent article according to claim 3, wherein the antioxidant is one or more vitamins selected from the group consisting of vitamin A group, vitamin B group, vitamin D group, vitamin E group, and vitamin K group.

5. The barrier agent for the absorbent article according to any one of claims 1-4, further comprising an emollient agent or immobilizing agent, or both.

6. The barrier agent for the absorbent article according to any one of claims 1-5, wherein the emollient agent is an oily substance which is liquid or semi-solid at 25°C.

7. The barrier agent for the absorbent article according to claim 6, wherein the emollient agent is a fatty acid glyceride.

8. The barrier agent for the absorbent article according to any one of claims 1-7, wherein the immobilizing agent has a melting point of 40-90°C and a surface tension of 25-45 dyn/cm at 20°C.

9. The barrier agent for the absorbent article according to claim 8, wherein the immobilizing agent is one or more compounds selected from the group consisting of a fatty acid glyceride, paraffin wax, and a higher alcohol.

10. An absorbent article to the surface of which a barrier agent of any one of claims 1-9 has been applied.
